# EUROPEAN PATENT APPLICATION

(11) **EP 2 975 458 A1**
(43) Date of publication of application: **20.01.2016**
(21) Application number: 14177267.3
(22) Date of filing: 16.07.2014
(51) Int. Cl.: G03B 42/02

(54) **Carrier for receiving an X-ray cassette, X-ray system and method for determining a size and/or orientation of an X-ray cassette**

(71) Applicant: Agfa HealthCare N.V., 2640 Mortsel (BE)
(72) Inventor: SCHINDLBECK, Günther, 81545 München (DE); ZEHETMAIER, Thomas, 85646 Neufarn (DE); NASER, Dr.Elian, 86316 Friedberg (DE); BENZINGER, Rupert, 85570 Markt Schwaben (DE); STALLMEISTER, Stefan, 85625 Glonn (DE); AUER, Franz, 84056 Rottenburg (DE); MINWEGEN, Heike, 83043 Bad Aibling (DE); GERSTLAUER, Bernd, 81541 München (DE)
(74) Representative: Linsmeier, Josef

(57) **Abstract**

The invention relates to a carrier (10) for receiving an X-ray cassette (15) having an X-ray detector, in particular an X-ray film, an X-ray storage phosphor plate or a solid-state X-ray detector, said carrier (10) comprising a support plate (11) for supporting the cassette (15); a first alignment device (20) for aligning and/or positioning the cassette (15) on the support plate (11) along an X axis of the support plate (11); and a first, in particular optical, sensor device (30) for sensing an X position of the cassette (15) with regard to the X axis. Moreover, the invention relates to an according X-ray system and a method for determining a size and/or orientation of an X-ray cassette (15) inserted into the carrier (10) according to the invention.

## Description

The present invention relates to a carrier for receiving an X-ray cassette, an X-ray system comprising such a carrier and a corresponding method for determining a size and/or orientation of an X-ray cassette inserted into such a carrier.

X-ray systems for medical applications typically comprise an X-ray source and an X-ray table or wall stand, to which a carrier, a so-called Bucky, carrying an X-ray cassette is attached. In typical imaging applications only a part of a body needs to be imaged, so that it is possible to reduce the amount of X-rays applied to the body by limiting the X-ray field to the body region to be imaged. Usually, the size of the respectively used X-ray cassettes depends on the dimensions of the body part to be imaged.

It is an object of the invention to provide a carrier for receiving an X-ray cassette, an X-ray system and a corresponding method allowing for simply and reliably determining a size and/or orientation of an X-ray cassette inserted into the carrier.

The object is achieved by a carrier according to claim 1, an X-ray system according to claim 11 and a method according to claim 12.

According to an aspect of the invention, a carrier for receiving an X-ray cassette having an X-ray detector comprises a support plate for supporting the cassette, a first alignment device for aligning and/or positioning the cassette on the support plate along an X axis of the support plate, and a first, particularly optical, sensor device for sensing an X position of the cassette with regard to the X axis.

According to another aspect of the invention, an X-ray system comprises a carrier for receiving an X-ray cassette according to the invention, an X-ray source, and a control device for controlling at least one of the first, second and third sensor devices and/or the X-ray source. The control device is adapted for determining a size and/or orientation of an inserted X-ray cassette based on sensor data received from at least one of the first, second and third sensor devices. The X-ray source is adapted for adapting a size of an X-ray field emitted by the X-ray source according to the determined size and/or orientation of the inserted X-ray cassette.

According to yet another aspect of the invention, a method for determining a size and/or orientation of an X-ray cassette inserted into the carrier according to the invention is provided, wherein a size and/or orientation of the cassette is determined by comparing an X dimension of the cassette sensed by the first sensor device and at least one Y dimension of the cassette sensed by at least one of the second and third sensor devices or calculated based on the sensed X dimension.

Within the context of the invention, the term "X-ray detector" may preferably relate to different types of media and/or devices being configured to detect and/or store X-ray radiation, in particular to a "classical" photographic X-ray film, a photostimulable storage phosphor plate being adapted for storing X-rays and emitting emission light upon stimulation with stimulation light, or a detector device being adapted for receiving X-rays and converting same into electrical signals, e.g. by means of a two-dimensional solid-state detector array.

Accordingly, the term "X-ray cassette having an X-ray detector" may preferably relate to a cassette-like housing being configured to receive and/or accommodated an X-ray film or a storage phosphor plate, or to a detector device having dimensions and/or a form which preferably correspond to the dimensions and/or the form of an X-ray cassette or a cassette-like housing for an X-ray film or a storage phosphor plate.

Aspects of the invention are based on the approach to provide a Bucky carrier with an aligning and/or a clamping mechanism being configured to align and/or clamp the X-ray cassette with respect to at least one direction, e.g. the X direction. Preferably, the aligning and/or a clamping mechanism is configured to center the X-ray detector automatically with respect to a center of the direction, e.g. the center of the X direction. Moreover, one or more, preferably optical, sensor devices are provided which are configured to sense the presence of the X-ray cassette at one or more X and/or Y positions along the X direction or Y direction, respectively. By combining the alignment, in particular a centering, and/or clamping, of the X-ray cassette inserted into the Bucky with a, preferably optical, sensing of the presence of the X-ray cassette at pre-defined sensor positions along the X direction and/or the Y direction, X and/or Y dimensions of the inserted X-ray cassette and/or its orientation can be derived and/or calculated automatically in a simple and reliable way.

Advantageously, sensor data by the, particularly optical, sensors of such a sensor device can be derived without mechanically interfering with the inserted cassette. Thereby, X-ray cassettes of all sizes can be inserted into the Bucky on its cassette support plate, just limited by the size of the support plate but not by the sensor devices. Moreover, sensor data derived from one sensor device can preferably be used for determining one dimension of the X-ray cassette and, preferably by comparison with dimension values stored in an X-ray cassette size database, also for indirectly determining another dimension of the X-ray cassette. Further, sensor data derived from two or more sensor devices can be used for determining both dimensions of rectangular shaped X-ray cassettes or for determining more dimensions of a differently shaped X-ray cassette. Thereby, the combination of sensor data from sensor devices for different directions of the X-ray cassette allows for determining the size and/or the orientation of the cassette.

In summary, the invention allows for a reliable and automatic determination of a size and/or orientation of an X-ray cassette inserted into a Bucky without the need of medical staff interference. Thereby, an automatic adaptation of an X-ray field applied to a patient by the X-ray system to the size and/or orientation of the inserted X-ray cassette is possible. This renders the radiographing process of a patient both time-saving and easy to handle while X-ray exposure of the patient is minimized.

According to a preferred embodiment, a sensor device according to the invention comprises one, two or a multitude of optical sensors, particularly charge-coupled devices (CCD), contact image sensors (CIS), photoelectric sensors, electrooptical sensors and/or optical position sensors (OPS).

According to another preferred embodiment of the invention, the first alignment device comprises a first X alignment element and a second X alignment element, wherein these alignment elements are particularly configured as or comprise a clamp and/or a slider element. The second X alignment element is coupled to the first X alignment element such that a movement of the first X alignment element along the X axis causes a movement of the second X alignment element along the X axis. Such a coupling allows for an easy and quick handling of the Bucky, especially when inserting, aligning and/or positioning an X-ray cassette, since only the first X alignment element has to be handled and the second X alignment element is moved automatically through the coupling.

According to another preferred embodiment of the invention, the first X alignment element and the second X alignment element are coupled such that a movement of the first X alignment element along the X axis causes a movement of the second X alignment element in the opposite direction along the X axis. Such a coupling can be provided by a, particularly scissor-like, so-called scissor or shear kinematics, which connects the first and the second X alignment element in a way that forces opposite movement along the X axis. This forced opposite movement may allow for an alignment and/or positioning of the X-ray cassette that is always centered to the same X direction position, wherein this X direction position is the position, to which both the first X alignment element and the second X alignment element have an equal X direction distance (due to being forced by the scissor kinematics). Thereby, sensing only one X position of, particularly an edge of, the cassette is sufficient for calculating the X dimension of the cassette, since the opposing edge of the cassette is forced to be at an equal distance of a reference zero X position (X=0) by the kinematics.

According to another preferred embodiment, the carrier comprises a second alignment device for aligning the cassette on the support plate along a Y axis of the support plate, and a second, particularly optical, sensor device for sensing a Y position of the cassette with regard to the Y axis. Thereby, a determination of a size and/or orientation of an X-ray cassette, based also on Y cassette position sensor data and thus very reliable (compared to deriving a Y dimension from a database based on sensor data of an X position of the cassette) is provided, particularly by combining sensor data from the first sensor device and the second sensor device.

According to another preferred embodiment of the invention, scissor or shear kinematics as described above for X alignment elements along the X axis can also be applied to Y alignment elements along the Y axis, allowing for according advantages.

According to another preferred embodiment of the invention, the carrier comprises a third alignment device for aligning the cassette on the support plate along the Y axis, and a third, particularly optical, sensor device for sensing another Y position of the cassette with regard to the Y axis. A combination of a second and a third aligning device (with their respective sensor devices) according to the invention allows for a flexible alignment and/or positioning of the X-ray cassette with respect to its Y position on the cassette support plate, since the second and the third aligning device allow for alignment and/or positioning independent of each other. Therefore, the X-ray cassette may be positioned at different Y positions, whilst the second and the third sensor device still allow for a determination of the positions of, particularly two opposing edges, of the X-ray cassette and thereby a determination and/or calculation of its Y dimension.

According to yet another preferred embodiment of the invention, the first sensor device comprises at least two, particularly optical, sensors, particularly a sensor strip, panel or bar with sensors, at different positions along an X axis dimension of said sensor device. Such a sensor device allows for reading out signals from different X axis positions of the cassette support plate in a simple, quick and/or cheap way.

According to another preferred embodiment of the invention, at least one of the second and third sensor devices comprises at least two, particularly optical, sensors, particularly a sensor strip, panel or bar with sensors, at different positions along a Y axis dimension of said at least one sensor device. Such a sensor device allows for reading out signals from different y axis positions of the cassette support plate in a simple, quick and/or cheap way.

According to another preferred embodiment, the sensors of at least one of the sensor devices of the invention comprise charge-coupled devices (CCD), contact image sensors (CIS), photoelectric sensors, electro-optical sensors and/or optical position sensors (OPS).

According to another preferred embodiment of the invention, sensors of at least one of the first, second and third sensor devices are arranged in an X-Y-plane being parallel or coplanar to the cassette support plate. Thereby, the sensor devices can be assembled at a position on or below the surface of the cassette support plate, where the position allows for good detection of the presence of the cassette without interfering with the insertion process of the cassette into the Bucky.

According to another preferred embodiment of the invention, sensors of at least one of the first, second and third sensor devices are adapted for sensing the presence of the cassette at the position of the respective sensor. This allows for a simple, quick and/or automated determination of a size and/or an orientation of the X-ray cassette.

According to yet another preferred embodiment of the invention, the carrier comprises a control device for controlling at least one of the first, second and third sensor devices. The control device is adapted for determining a size and/or orientation of the cassette by comparing an X dimension of the cassette sensed by the first sensor device and at least one Y dimension of the cassette that is sensed by at least one of the second and third sensor devices or that is calculated based on the determined X dimension. Thereby, an automatic adaptation of an X-ray field applied to a patient by the X-ray system to a maximum of the size and/or orientation of the inserted X-ray cassette is possible by considering the determined size and/or orientation of the cassette. This allows for time-saving and easy handling of a patient scanning process which is at the same time determined to minimizing X-ray exposure of the patient.

According to a preferred embodiment, an X and/or Y position of a sensor of a sensor device is predetermined by the calculation of the distance of the position of the sensor from a predetermined zero X and/or zero Y position (X = 0 and/or Y=0), wherein the predetermined zero X and/or Y position is preferably defined by a center Y and/or X axis of the cassette support plate or by a Y and/or X edge of the cassette support plate. Thereby, sensor data representative of the presence or the absence of an X-ray cassette at the position of the sensor of the sensor device can be used for calculating an X and/or Y dimension of the X-ray cassette.

According to a preferred embodiment of a method according to the invention, an outermost sensor covered or an innermost sensor not covered by the cassette is determined for the first sensor device, an X dimension of the cassette is calculated based on the X position of this sensor, and a Y dimension of the cassette is calculated based on a determination of a Y position of an outermost sensor covered or an innermost sensor not covered by the cassette for the second sensor device and/or based on a determination of a Y position of an outermost sensor covered by the cassette for the third sensor device. In this way, the invention of the X-ray cassette can be calculated in a reliable, quick and/or automated way.

According to another preferred embodiment of a method according to the invention, the X dimension of the cassette is determined by doubling the X position determined by the first sensor device, and/or the Y dimension of the cassette is determined by adding up the Y position determined for the second sensor device and the Y position determined for the third sensor device. This allows for a sensor-based calculation of a size and/or an orientation of the X-ray cassette, particularly in a carrier comprising only one (first) X alignment device with coupled X alignment elements and comprising two (second and third) Y alignment devices for flexible Y positioning of the X-ray cassette.

According to yet another preferred embodiment of a method according to the invention, an X dimension and/or Y dimension is determined by comparing a dimension value of at least one of the first, second and third sensor devices with predetermined cassette dimensions and selecting one of the predetermined cassette dimension, particularly using the control device. Preferably, the predetermined cassette dimensions are stored within a database that can be accessed by the control device, particularly in order to compare X or Y dimension values calculated from determined sensor values or to compare determined sensor values themselves to the cassette dimensions stored within the database. By performing such a comparison, the size and/or orientation of X-ray cassettes usually used for an X-ray system can be derived from the raw sensor values, which may in certain cases differ from the actual cassettes dimension (in X and/or Y direction) in case there is a distance in between the cassette's edge and the position of the sensor. By comparing the measured sensor value with the storage database value, a certain stored cassette size and/or orientation can be allocated for example for a certain outermost covered or innermost not covered sensor of a certain sensor device, or for a certain range of measured sensor values.

Further advantages, features and examples of the present invention will be apparent from the following description of following figures, showing:
- Fig. 1 a-b: an X-ray table with an example of a carrier for receiving an X-ray cassette in different views (top, side) of a schematic representation;
- Fig. 2: an X-ray table with another example of a carrier for receiving an X-ray cassette in a schematic top view;
- Fig. 3: the X-ray table of Fig. 2 with an inserted X-ray cassette in a schematic top view;
- Fig. 4: another example of a carrier for receiving an X-ray cassette with an X-ray cassette inserted in a first orientation in a schematic top view;
- Fig. 5: the carrier of Fig. 4 with the X-ray cassette inserted in a second orientation in a schematic top view;

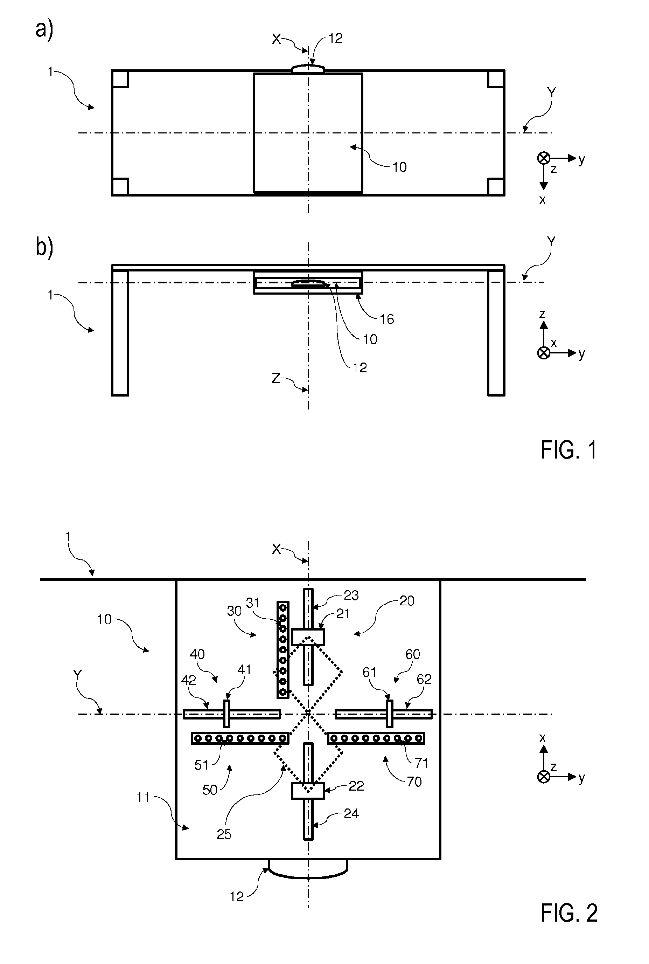

Fig. 1 shows a top view (a) and a side view (b) of an X-ray table 1 with an example of a carrier 10 for receiving an X-ray cassette (not shown) in a schematic representation. On the lower side of the table top of the X-ray table 1 a holding structure 16 is provided for receiving the Bucky type X-ray cassette carrier 10 in a drawer-like manner. On the carrier 10 a handle or grip 12 is provided which facilitates pulling and pushing the carrier 10 out of and into the holding structure 16.

Fig. 2 shows a detail of an X-ray table 1 with another example of a carrier 10 for receiving an X-ray cassette (not shown) in a schematic top view. The carrier 10, in particular cassette support plate 11 of the carrier 10, can be pulled out of the carrier holding structure (not shown) parallel to an X direction. The surface of cassette support plate 11 extends within an X-Y-plane defined by axes parallel to the transverse axis X of the X-ray table top and to the longitudinal axis Y of the X-ray table top (see also Fig. 1).

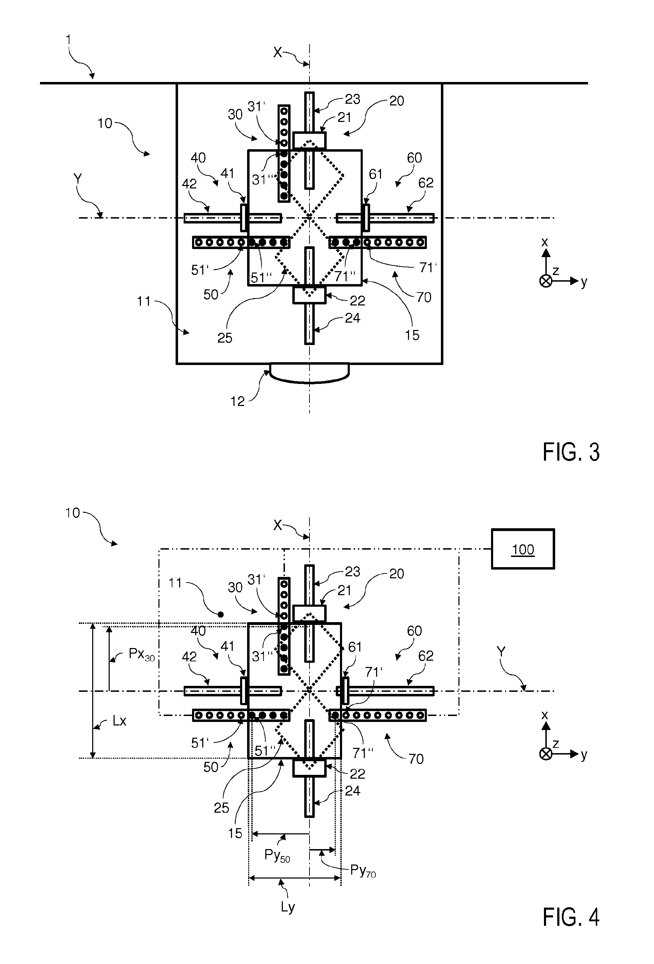

Fig. 3 shows the example of the X-ray table 1 of Fig. 2 with an inserted X-ray cassette 15 in the same schematic top view.

Carrier 10 (see also Fig. 2) comprises a first alignment device 20 with a first X alignment element 21 formed as a clamp and a second X alignment element 22 also formed as a clamp. The first X alignment element 21 is force-guided by an X alignment rail 23 with respect to movement along the X axis; the second X alignment element 22 is force-guided by another X alignment rail 24 with respect to movement along the X axis.

X alignment clamps 21 and 22 are coupled to each other by a scissor kinematics assembly such that a movement of first X alignment clamp 21 in negative X direction forces second X alignment clamp 22 to perform the same amount of movement in the opposite positive X direction, each of them guided by the respective rail 23 or 24. Thereby it is ensured that an inserted X-ray cassette is centered at the depicted Y axis on the cassette support plate 11 with respect to X direction when being aligned and/or positioned.

First alignment device 20 preferably also comprises a first sensor device 30 with a sensor bar having a given number, e.g. nine, sensors 31 spaced apart from each other with respect to X direction. Thus, along the possible positions of the first X alignment clamp 21, particularly the position of its inner edge aligning and/or positioning a corresponding edge of the cassette 15, sensors 31 are positioned and adapted to sense the presence or absence of a cassette 15 at their respective position.

Since the positions of the first X alignment clamp 21 and the second X alignment clamp 22 are preferably symmetrical with respect to the Y axis, an X dimension of an inserted cassette 15 can be calculated by determining an X position of only one edge of the cassette, in particular by doubling the determined X position.

In the exemplary embodiment depicted in Figs. 2 and 3, a presence of the cassette 15 is detected by sensor 31" and four other sensors 31, each of them being closer to the Y axis then sensor 31", as being present at the position of the respective sensor 31. Thus, sensor 31" is the outermost sensor detecting the presence of a cassette 15 at its position.

Carrier 10 also comprises a second alignment device 40 with a Y alignment element 41 formed as a slider for aligning and/or positioning the cassette 15 with respect to a positive Y direction. Y alignment slider 41 is guided by a Y alignment rail 42. Furthermore, carrier 10 also comprises a third alignment device 60 with another Y alignment element 61 formed as a slider for aligning and/or positioning the cassette 15 with respect to a negative Y direction. Y alignment slider 61 is guided by a Y alignment rail 62.

Applying both Y alignment sliders 41 and 61 allows the X-ray medical staff to flexibly position the X-ray cassette with respect to both Y directions, meaning flexibility of putting the X-ray cassette and thus the scanned body region further to the front or to the rear of the X-ray table, e.g. further to the head or to the legs of the patient scanned.

Second alignment device 40 also comprises a second sensor device 50 with a sensor bar having nine sensors 51 spaced apart from each other with respect to Y direction. Thus, along the possible positions of Y alignment slider 41, particularly at the position of its inner edge aligning and/or positioning a corresponding edge of the cassette, sensors 51 are positioned and adapted for sensing the presence or absence of the cassette 15 at their respective position. Cassette 15 is detected by sensor 51 "and three other sensors 51, each of them closer to the X axis than sensor 51 ", as being present at the position of the respective sensor 51.

Third alignment device 60 also comprises a third sensor device 70 with a sensor bar having a number of, e.g. nine, sensors 71 spaced apart from each other with respect to Y direction. Thus, along the possible positions of Y alignment slider 61, particularly at the position of its inner edge aligning and/or positioning a corresponding edge of the cassette, sensors 71 are positioned and adapted to sense the presence or absence of the cassette 15 at their respective position. Cassette 15 is detected by sensor 71" and two other sensors 71, each of them being closer to the X axis than sensor 71 ", as being present at the position of the respective sensor 71.

Since the positions of the second Y alignment slider 41 and the third Y alignment slider 61 can be adjusted independently from each other, a Y dimension of an inserted cassette 15 can be calculated by determining a Y position of a left (as depicted in Fig. 3) edge of the cassette 15 using the sensors 51 of the second sensor device 50 and determining a Y position of the right edge of the cassette 15 using the sensors 71 of the third sensor device 70. The Y dimension of the inserted cassette 15 then this calculated by addition of the distances between the X axis as Y=0 reference and outermost sensor 51 "on the one (left) side and outermost sensor 71" on the other (right) side.

Fig. 4 shows another example of a carrier 10 for receiving an X-ray cassette with an X-ray cassette 15 inserted in a first orientation in a schematic top view.

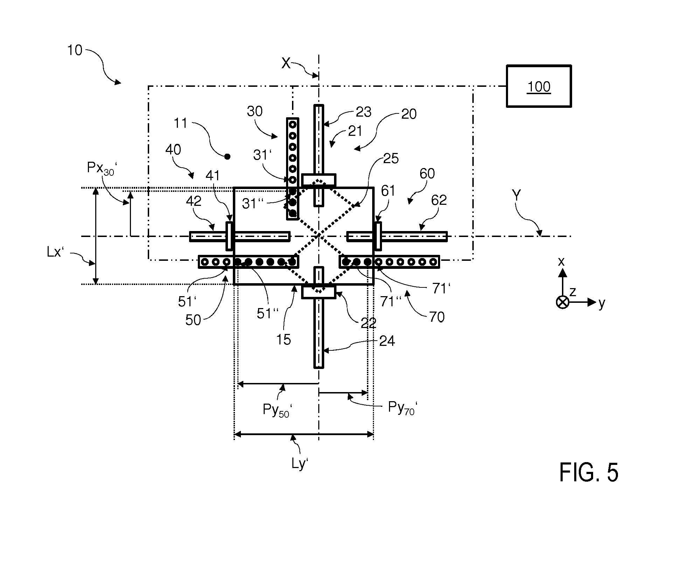

Fig. 5 shows the carrier 10 of figure 4 with the X-ray cassette 15 inserted in a second orientation in a schematic top view.

The carrier 10 (see Figs. 4 and 5) preferably comprises a control device 100 that is connected to the first, second and third sensor devices 30, 50 and 70, particularly for receiving sensor data and/or sending control commands.

The control device 100 is preferably also connected to a database (not shown) in which predefined cassette 15 sizes - particularly sizes of various cassette types used in the present X-ray system - are stored, and to the X-ray source (not shown) of the X-ray system.

X and Y dimensions of cassette 15 inserted into carrier 10 and aligned and/or positioned by the first, second and third alignment devices 20, 40 and 60 can be determined in the way described above (see description of the examples given in figures 2 and 3).

However, there may be small differences between the X/Y positions Px₃₀, Py₅₀ and/or Py₇₀ determined by sensor devices 30, 50 or 70 and the actual dimension Lx and/or Ly of the cassette 15 with respect to X/Y direction, since sensor devices 30, 50 and 70 only deliver discrete position values, depending on which of the respective sensors of the sensor device 30, 50 and 70 is the outermost sensor covered by the cassette 15. For example, the edge of said cassette 15 may be placed somewhere between the outermost covered sensor 31", 51" and 71", respectively, and an innermost uncovered sensor 31', 51' and 71', respectively. In such cases, the accuracy of the sensor-based determination of X and Y dimensions of the cassette 15 described above is limited to the distance between two sensors 31 (X dimension) or 51 and 71 (Y dimension) of a sensor device 30 or 50 and 70, respectively.

In order to provide an even more exact determination of the X and Y dimensions of a cassette, control device 100 preferably compares each of the values, i.e. (2 x Px₃₀) and (Py₅₀ + Py₇₀) corresponding to the determined X and Y dimension, respectively, of the cassette 15 with corresponding predefined cassette sizes stored in the database, e.g. Lx = 35mm, Lx=43mm , Ly=35, Ly=43mm, and determines the size of the cassette 15 by determining the database values Lx, Ly which are closest to the values of the determined X and Y dimension.

In this way, also a determination of the orientation of the cassette 15 is effectuated, since the orientation is determined by whether the determined Lx value is larger or smaller than (or equal to) the determined Ly value. In case the Lx value is larger than the Ly value, an orientation value "landscape" is allocated, while in case the Ly value is larger than the Lx value, an orientation value "portrait" is allocated.

In an alternative configuration of control device 100, each sensor 31, 51 and 71 is may be assigned to a predetermined cassette size or X and Y dimension. Such a configuration can be useful in case where only a small and/or predetermined number of possible cassette sizes are used, e.g. two, three or four cassette sizes. The cassette size in this case is preferably determined by a combination of the values of each outermost covered sensor 31 ", 51 ", 71" of all three sensor devices 30, 50, 70.

Preferably, the control device 100 is configured to control the X-ray source of the X-ray system in consideration of the determined cassette size and/or orientation such that only an X-ray field corresponding to the determined size and/or position of the cassette 15 is exposed to X-rays.

### Reference signs

- 1: X-ray table

- 10: bucky (X-ray cassette carrier)
- 11: cassette support plate
- 12: bucky grip (handle)
- 15: cassette/X-ray detector
- 16: holding structure

- 20: first alignment device
- 21: X alignment clamp (X direction alignment element)
- 22: X alignment clamp (X direction alignment element)
- 23: X alignment rail
- 24: X alignment rail
- 25: scissor assembly (X alignment coupling)
- 30: first sensor device
- 31: optical sensors

- 40: second alignment device
- 41: Y alignment slider (Y direction alignment element)
- 42: Y alignment rail
- 50: second sensor device
- 51: optical sensors

- 60: third alignment device
- 61: Y alignment slider (Y direction alignment element)
- 62: Y allignment rail
- 70: third sensor device
- 71: optical sensors
- 100: control device

- X: lateral axis of the X-ray table / first axis of the support plate
- Y: longitudinal axis of the X-ray table / second axis of the support plate
- Z: vertical axis of the X-ray table

- Lx: dimension of the cassette along the X axis
- Ly: dimension of the cassette along the Y axis

- Px: X position of a sensor
- Py: Y position of a sensor

## Claims

1. Carrier (10) for receiving an X-ray cassette (15) having an X-ray detector, in particular an X-ray film, an X-ray storage phosphor plate or a solidstate X-ray detector, said carrier (10) comprising
- a support plate (11) for supporting the cassette (15),
- a first alignment device (20) for aligning and/or positioning the cassette (15) on the support plate (11) along an X axis of the support plate (11), and
- a first, in particular optical, sensor device (30) for sensing an X position (Px₃₀) of the cassette (15) with regard to the X axis.

2. Carrier (10) according to claim 1, the first alignment device (20) comprising a first X alignment element (21) and a second X alignment element (22) coupled to the first X alignment element (21) such that a movement of the first X alignment element (21) along the X axis causes a movement of the second X alignment element (22) along the X axis.

3. Carrier (1) according to claim 1 or 2, the first X alignment element (21) and the second X alignment element (22) being coupled such that a movement of the first X alignment element (21) in a direction along the X axis causes a movement of the second X alignment element (22) in the opposite direction along the X axis.

4. Carrier (10) according to one of the preceding claims, the carrier (10) comprising
- a second alignment device (40) for aligning the cassette (15) on the support plate (11) along a Y axis of the support plate (11),
- a second, in particular optical, sensor device (50) for sensing a Y position (Py₅₀) of the cassette (15) with regard to the Y axis.

5. Carrier (10) according to one of preceding claims, the carrier (10) comprising
- a third alignment device (60) for aligning the cassette (15) on the support plate (11) along the Y axis,
- a third, in particular optical, sensor device (70) for sensing another Y position (Py₇₀) of the cassette (15) with regard to the Y axis.

6. Carrier (10) according to one of the preceding claims, the first sensor device (30) comprising at least two, in particular optical, sensors (31) being located at different positions along an X axis dimension of said sensor device (30).

7. Carrier (10) according to one of the preceding claims, at least one of the second and third sensor devices (50; 70) comprising at least two, particularly optical, sensors (51 ;71) at different positions along a Y axis dimension of said at least one sensor device (50; 70).

8. Carrier (10) according to one of the preceding claims, the sensors (31; 51; 71) of at least one of the first, second and third sensor device (30; 50; 70) being arranged in an X-Y-plane being parallel or coplanar to the cassette support plate (11).

9. Carrier (10) according to one of the preceding claims, the sensors (31; 51; 71) of at least one of the first, second and third sensor devices (30; 50; 70) being adapted for sensing the presence of the cassette (15) at the position (Px30; Py50; Py70) of the respective sensor (31; 51; 71).

10. Carrier (10) according to one of the preceding claims comprising a control device (100) being adapted to control at least one of the first, second and third sensor devices (30;50,70) and to determine a size (Lx; Ly) and/or an orientation of the cassette (15) by comparing
- an X dimension of the cassette (15) sensed by the first sensor device (30) and
- at least one Y dimension of the cassette (15) sensed by at least one of the second and third sensor devices (50; 70) or calculated based on the determined X dimension.

11. X-ray system comprising
- the carrier (10) for receiving an X-ray cassette (15) according to one of the preceding claims,
- an X-ray source,
- a control device (100) for controlling at least one of the first, second and third sensor devices (30; 50; 70) and/or the X-ray source,
the control device (100) being adapted to determine a size (Lx; Ly) and/or an orientation of an inserted X-ray cassette (15) based on sensor data received from at least one of the first, second and third sensor devices (30; 50; 70), and
the X-ray source being adapted to adapt a size of an X-ray field emitted by the X-ray source according to the determined size (Lx; Ly) and/or orientation of the inserted X-ray cassette (15).

12. Method for determining a size (Lx; Ly) and/or an orientation of an X-ray cassette (15) inserted into the carrier (10) according to one of the preceding claims, wherein
a size (Lx; Ly) and/or an orientation of the cassette (15) is determined by comparing
- an X dimension of the cassette (15) sensed by the first sensor device (30) and
- at least one Y dimension of the cassette (15) sensed by at least one of the second and third sensor devices (30; 50) or calculated based on the sensed X dimension.

13. Method according to the preceding claim, wherein
- an outermost sensor (31") covered by the cassette (15) is determined for the first sensor device (30),
- an X dimension (Lx) of the cassette (15) is calculated based on the X position (Px₃₀) of the determined outermost sensor (31") of the first sensor device (30), and
- a Y dimension (Ly) of the cassette (15) is calculated based on a determination of a Y position (Py₅₀) of an outermost sensor (51") covered by the cassette (15) for the second sensor device (50) and/or based on a determination of a Y position (Py₇₀) of an outermost sensor (71") covered by the cassette (15) for the third sensor device (70).

14. Method according to the preceding claim, wherein
- the X dimension (Lx) of the cassette is determined by doubling the X position (Px₃₀) determined by the first sensor device (30), and/or
- the Y dimension (Ly) of the cassette (15) is determined by adding up the Y position (Py₅₀) determined for the second sensor device (50) and the Y position (Py₇₀) determined for the third sensor device (70).

15. Method according to one of the claims 12 to 14, wherein
- an X dimension (Lx) and/or Y dimension (Ly) of the cassette is determined by comparing a dimension value of at least one of the first, second and thirst sensor devices (30; 50; 70) with predetermined cassette dimensions and selecting one of the predetermined cassette dimensions.
